# EUROPEAN PATENT SPECIFICATION

(11) **EP 0 871 456 B1**
(45) Date of publication and mention of the grant of the patent: **12.02.2003**
(21) Application number: 96912783.6
(22) Date of filing: 15.04.1996
(51) Int. Cl.: A61K 31/785, C08G 69/10

(54) **PHARMACEUTICAL COMPOSITIONS COMPRISING SYNTHETIC PEPTIDE COPOLYMER FOR PREVENTION OF GVHD**
PHARMAZEUTISCHE ZUSAMMENSETZUNGEN, DIE SYNTHETISCHE PEPTIDCOPOLYMERISATE ENTHALTEN, ZUR VERHÜTUNG VON GVHD
COMPOSITIONS PHARMACEUTIQUES COMPORTANT UN COPOLYMERE PEPTIDIQUE DE SYNTHESE ET SERVANT A LA PREVENTION DES TROUBLES DUS A LA REACTION GREFFE CONTRE HOTE

(30) Priority: 14.04.1995 US 421412; 06.10.1995 US 540388
(43) Date of publication of application: 21.10.1998
(73) Proprietor: YEDA RESEARCH AND DEVELOPMENT CO. LTD., Rehovot 76100 (IL); THE BOARD OF TRUSTEES OF THE LELAND STANFORD JUNIOR UNIVERSITY, Palo Alto, CA 94304-1850 (US)
(72) Inventor: AHARONI, Rina, 76100 Rehovot (IL); ARNON, Ruth, 76100 Rehovot (IL); CHAO, Nelson, J., Menlo Park, CA 94025 (US); SCHLEGEL, Paul, G., D-78112 St. Georgen (DE); SELA, Michael, 76100 Rehovot (IL); TEITELBAUM, Dvora, 76209 Rehovot (IL)
(74) Representative: VOSSIUS & PARTNER
(86) International application number: PCT/US96/05215
(87) International publication number: WO 96/032119

(56) References cited:
- WO-A-95/31990
- US-A- 3 849 550
- BLOOD 84 (8). 1994. 2802-2810, XP000577169 SCHLEGEL P G ET AL: "Prevention of graft - versus - host disease by peptide binding to class II major histocompatibility complex molecules." cited in the application
- PROCEEDINGS OF THE NATIONAL ACADEMY OF SCIENCES OF THE UNITED STATES OF AMERICA 91 (11). 1994. 4872-4876, XP002009925 FRIDKIS-HARELI M ET AL: "Direct binding of myelin basic protein and synthetic copolymer 1 to class II major histocompatibility complex molecules on living antigen-presenting cells-specificity and promiscuity." cited in the application
- 37TH ANNUAL MEETING OF THE AMERICAN SOCIETY OF HEMATOLOGY, SEATTLE, WASHINGTON, USA, DECEMBER 1-5, 1995. BLOOD 86 (10 SUPPL. 1). 1995. 224A, XP000578001 SCHLEGEL P G ET AL: "Inhibition of allorecognition and prevention of graft-vs-host disease ( GVHD ) by GLAT, a synthetic polymer with promiscuous binding to murine and human MHC class II molecules."

### SEQUENCE LISTING

### (1) GENERAL INFORMATION:

(i) APPLICANT:
   (A) NAME: Yeda Research and Development Co. Ltd.
   (B) STREET: P.O. Box 95
   (C) CITY: Rehovot
   (E) COUNTRY: Israel
   (F) POSTAL CODE (ZIP): 76100
   (G) TELEPHONE: 011-972-8-470617
   (H) TELEFAX: 011-972-8-470739

   (A) NAME: Board of Trustees of the Leland Stanford Junior University
   (B) STREET: 900 Welch Road, Suite 350
   (C) CITY: Palo Alto
   (D) STATE: California
   (E) COUNTRY: United States of America
   (F) POSTAL CODE (ZIP): 94304-1850
   (G) TELEPHONE: (415) 723-0651
   (H) TELEFAX: (415) 725-7295
(ii) TITLE OF INVENTION: PHARMACEUTICAL COMPOSITIONS COMPRISING SYNTHETIC PEPTIDE COPOLYMER FOR PREVENTION OF GVHD
(iii) NUMBER OF SEQUENCES: 6
(iv) CORRESPONDENCE ADDRESS:
   (A) ADDRESSEE: BROWDY AND NEIMARK
   (B) STREET: 419 Seventh Street N.W., Ste. 300
   (C) CITY: Washington
   (D) STATE: D.C.
   (E) COUNTRY: United States of America
   (F) ZIP: 20004
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.30
(vi) CURRENT APPLICATION DATA:
   (A) APPLICATION NUMBER:
   (B) FILING DATE:
   (C) CLASSIFICATION:
(vii) PRIOR APPLICATION DATA:
   (A) APPLICATION NUMBER: US 08/421,412
   (B) FILING DATE: 14-APR-1995
(vii) PRIOR APPLICATION DATA:
   (A) APPLICATION NUMBER: US 08/540,388
   (B) FILING DATE: 06-OCT-1995
(viii) ATTORNEY/AGENT INFORMATION:
   (A) NAME: BROWDY, Roger L.
   (B) REGISTRATION NUMBER: 25,618
   (C) REFERENCE/DOCKET NUMBER: AHARONI=1A PCT
(ix) TELECOMMUNICATION INFORMATION:
   (A) TELEPHONE: (202) 628-5197
   (B) TELEFAX: (202) 737-3528

### (2) INFORMATION FOR SEQ ID NO:1:

(i) SEQUENCE CHARACTERISTICS:
   (A) LENGTH: 11 amino acids
   (B) TYPE: amino acid
   (C) STRANDEDNESS: single
   (D) TOPOLOGY: linear
(ii) MOLECULE TYPE: peptide
(iii) FEATURE INFORMATION: The alanine at position 1 is acetylated
(xi) SEQUENCE DESCRIPTION: SEQ ID NO:1:
   Ala Ser Gln Ala Arg Pro Ser Gln Arg His Gly
   1               5                   10

### (2) INFORMATION FOR SEQ ID NO:2:

(i) SEQUENCE CHARACTERISTICS:
   (A) LENGTH: 13 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:2:
   Thr Gly Ile Leu Asp Ser Ile Gly Arg Phe Phe Ser Gly
   1               5                   10

### (2) INFORMATION FOR SEQ ID NO:3:

(i) SEQUENCE CHARACTERISTICS:
   (A) LENGTH: 14 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:3:
   Lys Met Lys Met Val His Ala Ala His Ala Lys Met Lys Met
   1               5                   10

### (2) INFORMATION FOR SEQ ID NO:4:

(i) SEQUENCE CHARACTERISTICS:
   (A) LENGTH: 13 amino acids
   (B) TYPE: amino acid
   (C) STRANDEDNESS: single
   (D) TOPOLOGY: linear
(ii) MOLECULE TYPE: peptide
(xi) SEQUENCE DESCRIPTION: SEQ ID NO:4:
   Val His Phe Phe Lys Asn Ile Val Thr Pro Arg Thr Pro
   1               5                   10

### (2) INFORMATION FOR SEQ ID NO:5:

(i) SEQUENCE CHARACTERISTICS:
   (A) LENGTH: 21 base pairs
   (B) TYPE: nucleic acid
   (C) STRANDEDNESS: single
   (D) TOPOLOGY: linear
(ii) MOLECULE TYPE: cDMA
(xi) SEQUENCE DESCRIPTION: SEQ ID NO:5:
   CCAAGCTTCC TTGTGCAAGT A 21

### (2) INFORMATION FOR SEQ ID NO:6:

(i) SEQUENCE CHARACTERISTICS:
   (A) LENGTH: 20 base pairs
   (B) TYPE: nucleic acid
   (C) STRANDEDNESS: single
   (D) TOPOLOGY: linear
(ii) MOLECULE TYPE: cDNA
(xi) SEQUENCE DESCRIPTION: SEQ ID NO:6:
   AGCCCAAAGT CCATCAGTGG 20

## Description

### FIELD OF THE INVENTION

The present invention relates to organ transplantation in humans in general, and more specifically to prevention of graft-versus-host-disease, particularly in bone marrow transplantation.

### BACKGROUND OF THE INVENTION

Bone marrow transplantation (BMT) is increasingly used in humans for treatment of many life-threatening hematologic disorders, especially leukemia and certain immune deficiency diseases. However, graft-versus-host disease (GVHD) remains the major barrier for effective allogeneic bone marrow transplantation. This complication occurs in approximately 30% of bone marrow recipients. Up to half of those patients who develop GVHD may succumb to this process. This high morbidity and mortality has led to continuous interest in the possibility of controlling or preventing

### GVHD.

Current available approaches for prevention of GVHD include the use of non-specific immunosuppressive drugs, such as cyclosporine, methotrexate and/or prednisone. However, this treatment induces severe side effects, including nephrotoxicity, hypertension, hypercholesterolemia, diabetogenic effects, neurotoxicity, hirsutism and gengival hyperplasia. Moreover, the unselective depression of the entire immune system renders patients vulnerable to infections. In addition to the chronic administration of toxic, immunosuppressive agents, successful human allogeneic bone marrow transplantation depends, at the moment, on the availability of a suitable histocompatibility donor.

Clinicopathologically, two forms of GVHD have been recognized. Acute GVHD develops within the first 3 months after BMT and features disorders of skin, liver and gastrointestinal tract. Chronic GVHD is a multiorgan autoimmune-like disease, emerging from 3 months up to 3 years post-transplantation and shares features common to naturally occurring autoimmune disorders, like systemic lupus erythematosus (SLE) and scleroderma.

GVHD is caused by the competent donor T cells reacting against minor histocompatibility antigens (mHAg) of the recipient, even when the donor and recipient are major histocompatibility complex (MHC)-identical. The donor T cells are sensitized to such alloantigens and then directly, or through secondary signals, attack the host cells. These mHAg display a tissue-specific distribution (Griem et al., 1991). Recent experimental evidence has clearly demonstrated that human (den Haan et al., 1995; Wang et al., 1995) and murine (Speiser, et al., 1990; Wallny et al., 1990; Roopenian, et al., 1993) mHAg correspond to recipient self-peptides presented by MHC class I and II molecules. This initial recognition process of host alloantigen after transplantation is further enhanced by the release of cytokines (Rus et al., 1995; Nestel et al., 1992) resulting in non-specific T cell activation and in the up-regulation and de novo expression of MHC molecules.

Novel experimental approaches towards prevention of murine GVHD have focused on the molecular basis of antigen recognition by T cells and on the early stages of T cell activation. These approaches aim at interference with the antigen-specific signal generated through the T cell receptor (Schlegel et al., 1994; Blazar et al., 1994) and at the inhibition of costimulatory signals (Schlegel et al., 1995). It has previously been demonstrated that small, synthetic peptides of 11-14 amino acids length with high binding affinity for specific class II MHC molecules are capable of preventing murine GVHD (Schlegel et al., 1994). This approach, however, has been limited by the need for allele-specificity of the inhibitor peptides (Schlegel et al., 1994) and by the difficulty of achieving sustained tissue levels of such low molecular weight peptides over a prolonged period of time after injection (Schlegel et al., 1994; Ishioka et al., 1994; Samson et al., 1995).

A high molecular weight synthetic basic random copolymer consisting of L-Ala, L-Glu, L-Lys and L-Tyr residues in the molar ratio of about 6 parts Ala to 2 parts Glu to 4.5 parts Lys to 1 part Tyr, and having a molecular weight of 15,000-25,000, was first described in US Patent No. 3,849,550 as an agent for treatment or prevention of experimental allergic encephalomyelitis (EAE), a disease resembling multiple sclerosis (MS) that can be induced in susceptible animals (Sela et al., 1990; Teitelbaum et al., 1971; Teitelbaum et al., 1973). Batches of this copolymer of average molecular weight 23,000, designated Copolymer 1 or Cop 1, were shown to be highly effective in protecting and suppressing EAE in several animal species (Teitelbaum et al., 1971, 1974a, 1974b). Later, Cop 1 was found to significantly reduce the number of relapses in patients with the exacerbating-remitting form of MS (Bornstein et al., 1990; Johnson et al., 1994). Subsequent studies have demonstrated the direct binding of Cop 1 to murine and human class II MHC molecules of different haplotypes (Fridkis-Hareli et al., 1994).

The mechanism underlying the therapeutic activity of Cop 1 in MS has been extensively studied. The interaction of Cop 1 with MHC molecules may result in two main and distinct underlying mechanisms for disease suppression, namely the induction of antigen specific suppressive regulatory cells (Teitelbaum et al., 1988) and competition with myelin antigens for activation of specific T cell responses (Fridkis-Hareli et al., 1995). Recent studies demonstrated that Cop 1 binds directly to class II molecules without the need for intracellular processing (Claman et al., 1985).

The first step essential for the activation of these specific processes is the binding of Cop 1 to the histocompatibility molecules. Indeed, it has been shown that a low molecular weight version of Cop 1 - two different batches of molecular weight 5,550 and 8,600, and relative molar ratio of L-Ala (4.1-5.8 residues), L-Glu (1.4-1.8 residues), L-Lys (3.2-4.2 residues) and L-Tyr (1 residue) - binds very efficiently to a variety of MHC class II molecules of mouse and human origin, and furthermore competes with myelin basic protein (MBP) and its major epitope p84-102 for MHC binding and can even displace such antigens that had already been bound to the MHC molecule (Fridkis-Hareli et al., 1994; WO 95/31990).

None of the prior art publications describes or suggests that any form of Cop 1 may be used to prevent or to treat GVHD.

### SUMMARY OF THE INVENTION

It has now been found in accordance with the present invention that a GLAT copolymer can be used successfully to prevent experimental graft-versus-host disease in an animal model which is similar to the matched bone marrow transplantation in humans. This murine system comprises bone marrow and spleen cells of B10.D2 mice transplanted to BALB/c recipients (both H-2d), but clearly different in minor histocompatibility antigens. The murine GVHD model of B10.D2/nSnJ → BALB/c (both H-2d) across minor histocompatibility barriers has been described in detail (Schlegel et al., 1995; Korngold et al., 1987; Hamilton 1987; Webb et al., 1990) and was selected since it represents a model which is very similar to MHC-matched bone marrow transplantation in humans (Schlegel et al., 1995).

The present invention thus relates, in one aspect, to a pharmaceutical composition for use in the prevention and treatment of graft-versus-host disease comprising as active ingredient a synthetic random copolymer of average molecular weight of about 4,000-12,000 consisting of glutamic acid, lysine, alanine and tyrosine residues, in a relative molar ratio of 1.4-2.1 parts of Glu to 3.2-4.2 parts of Lys to 4.0-6.0 parts of Ala to 1.0 part of Tyr, herein referred to as GLAT copolymer, together with physiologically acceptable carriers.

The GLAT copolymer used in the present invention has preferably an average molecular weight of about 5,500-10,000 Da, more preferably of 6,000-8,000 Da, and most preferably, of about 6,000 or of about 8,000.

The Glu, Lys, Ala and Tyr residues may all have the L-configuration (L-GLAT) or the D configuration (D-GLAT) or some of the residues have the L, and the others the D, configuration (DL-GLAT). In one preferred embodiment, L-GLAT is used in the invention.

Preferred molar ratios of the amino acid residues include the relative molar ratios 1.9 Glu to 4.7 Lys to 6.0 Ala to 1.0 Tyr, 1.7 Glu to 3.8 Lys to 4.9 Ala to 1.0 Tyr, and 1.8 Glu to 4.0 Lys to 6.0 Ala to 1.0 Tyr.

Although we have described above some preferred embodiments of the invention, it is to be understood that the present invention encompasses any synthetic random copolymer of Glu, Lys, Ala and Tyr, having a relative molar ratio of the amino acid residues and an average molecular weight as defined herein, including those forms of Cop 1 described in the literature that fall within the definition of the present invention.

Another aspect of the invention relates to the use of said GLAT copolymer for the manufacture of a medicament for prevention and treatment of graft-versus-host disease in a patient in the course of bone marrow and organ transplantation.

In a preferred embodiment, the GLAT copolymer is used according to the invention for prevention of graft-versus-host disease in allogeneic bone marrow transplantation, optionally together with other immunosuppressive agents.

### BRIEF DESCRIPTION OF THE FIGURES

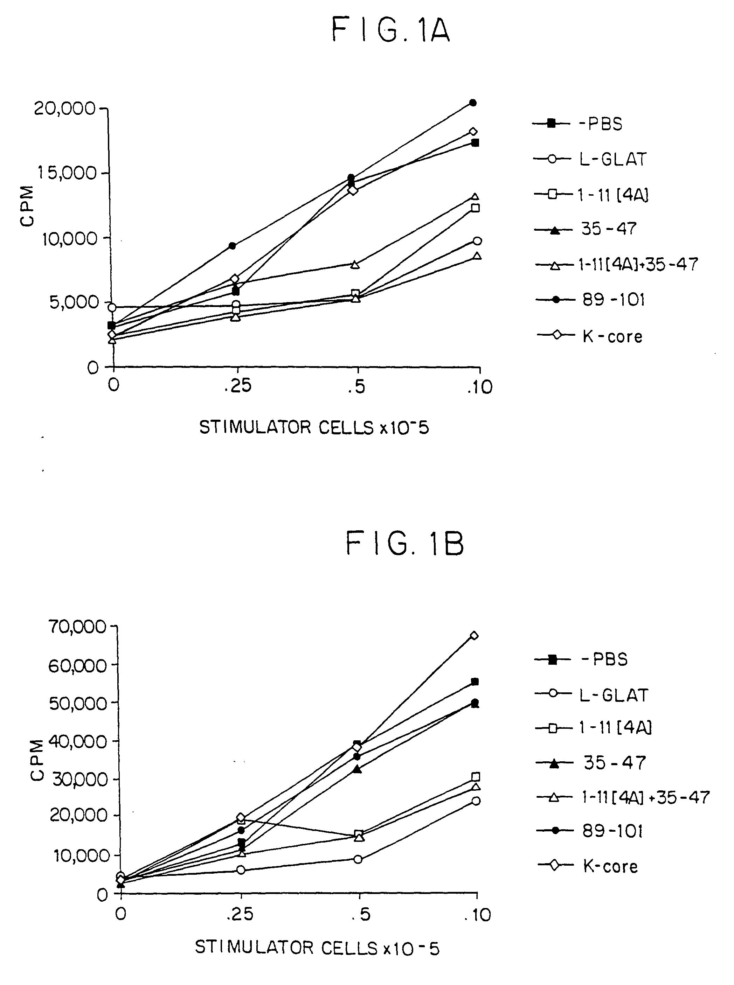

Figs. 1A-B show the inhibition of secondary mixed lymphocyte reaction (MLR) across minor as well as major histocompatibility barriers, caused by several peptides. The peptides L-GLAT (Batch I), MBP Ac1-11[4A], MBP 35-47, a mixture of MBP Ac1-11[4A] + MBP 35-47, MBP 89-101 and KM-core (all described in Materials and Methods hereinafter) were tested for their ability to inhibit the proliferation of B10.PL (H-2^{u}) mice responder cells to stimulator cells of either the same haplotype (PL/J mice, Fig. 1A) or of a different H-2 haplotype (BALB/c mice, Fig. 1B).

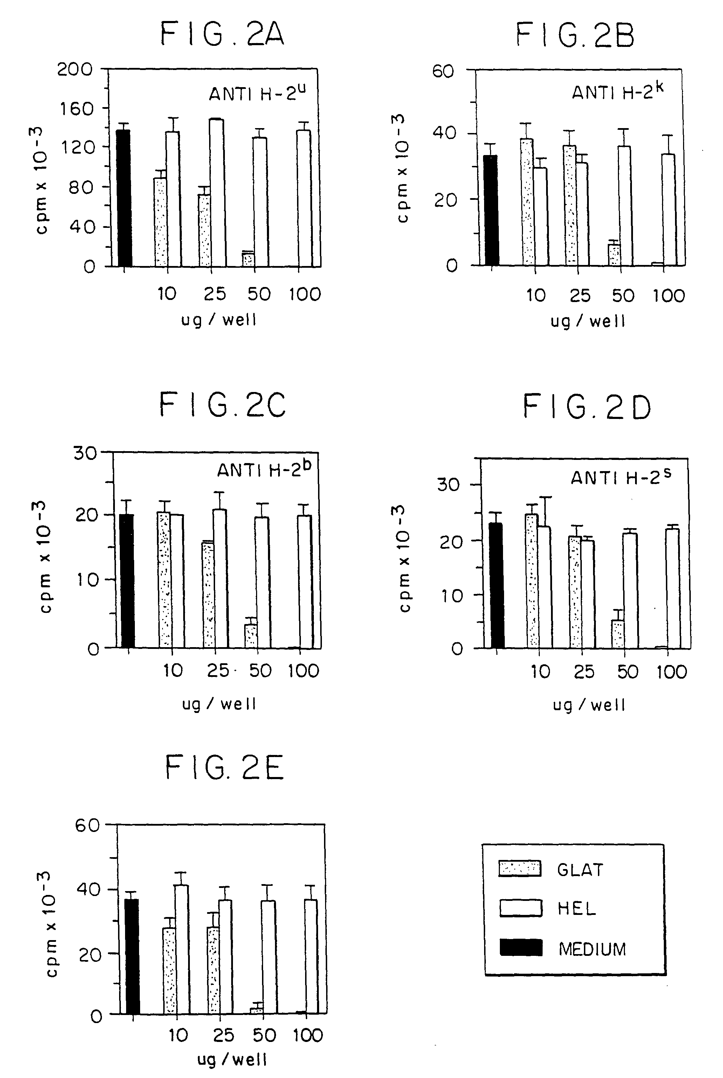

Figs. 2A-E are graphs showing dose-dependent inhibition of the MLC across major histocompatibility barriers. (Figs. 2A,B,C,D H-2^{d} anti H-2^{u,s,k,b} and E, H-2^{b} anti H-2^{d}). 2.5 × 10⁵ responder spleen cells were incubated with optional concentrations of irradiated (30 Gy, Cs source) spleen stimulator cells as described in Materials and Methods in a final volume of 200 µl. GLAT or HEL were added at the indicated concentrations (10-100 µg/well). After 96 hours of incubation, cultures were pulsed with 1 µCi [³H]-thymidine for an additional 16 hours. Similar degrees of inhibition by GLAT were observed using various amounts of stimulator cells (2.5, 5,0 or 10 × 10⁵, data not shown). No inhibition was observed with the addition of HEL in any of the strain combinations tested. Results represent one of five experiments with similar results.

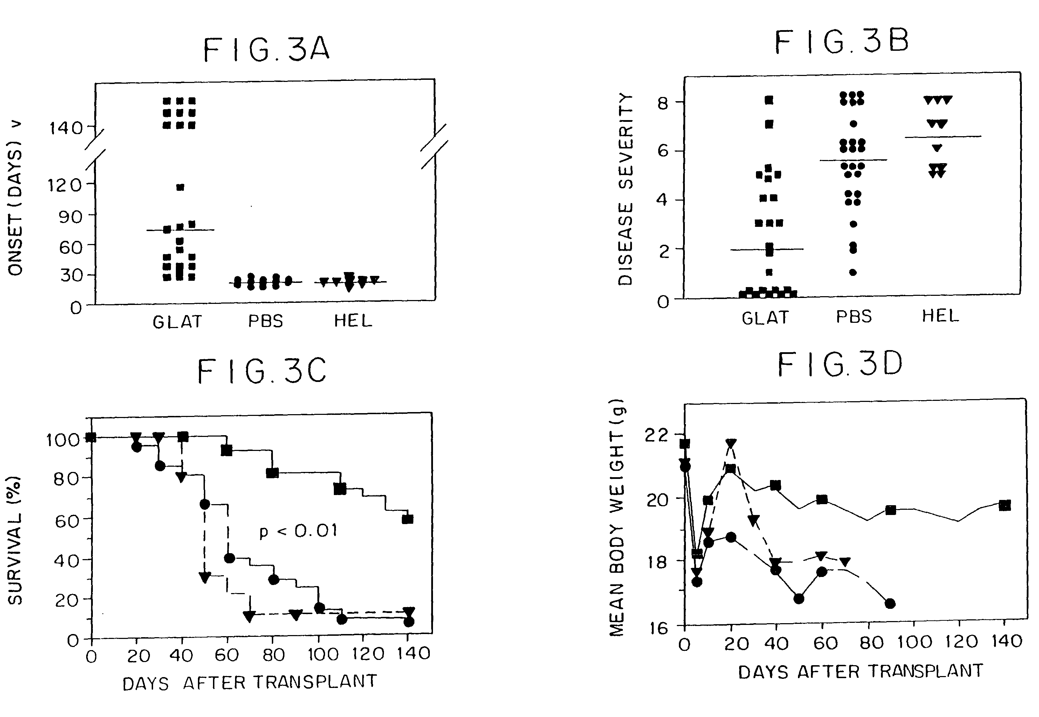

Figs. 3A-D are graphs showing the effect of treatment with GLAT on the induction of GVHD in B10.D2/nSnJ → BALB/c recipients (□ L-GLAT (Batch III), n=25; ○ PBS, n=26; ∇ HEL, n=10). Fig. 3A shows onset of GVHD in individual mice of the experimental groups. The median disease onset is indicated by lines (day 73/GLAT, day 21/PBS, and day 22/HEL). Statistical analysis by χ² distribution : Day 30 post-transplant : p < 0.001, incidence of GVHD in GLAT-treated mice (3/25) compared to the incidence in mice treated with PBS (26/26), or with HEL (10/10). Day 70 posttransplant : p < 0.001, incidence of GVHD in GLAT-treated mice (12/25) compared to PBS-treated (26/26), or p < 0.02, compared to HEL-treated mice (10/10). Fig. 3B shows maximum disease severity in individual mice of the experimental groups. The median disease severity is indicated by lines (2.0/GLAT, 5.5/PBS, 6.5/HEL). Fig. 3C shows actuarial survival from lethal GVHD. The median survival times (days) for the experimental groups are as follows: > 140 for GLAT, 47 for PBS, and 41 for HEL. Statistical analysis by Mann-Whitney test : p < 0.01, GLAT-treated mice compared to control mice with either PBS or HEL. Fig. 3D shows mean body weight curves after transplant. Data given in Figs. 3A-D are pooled from three similar experiments.

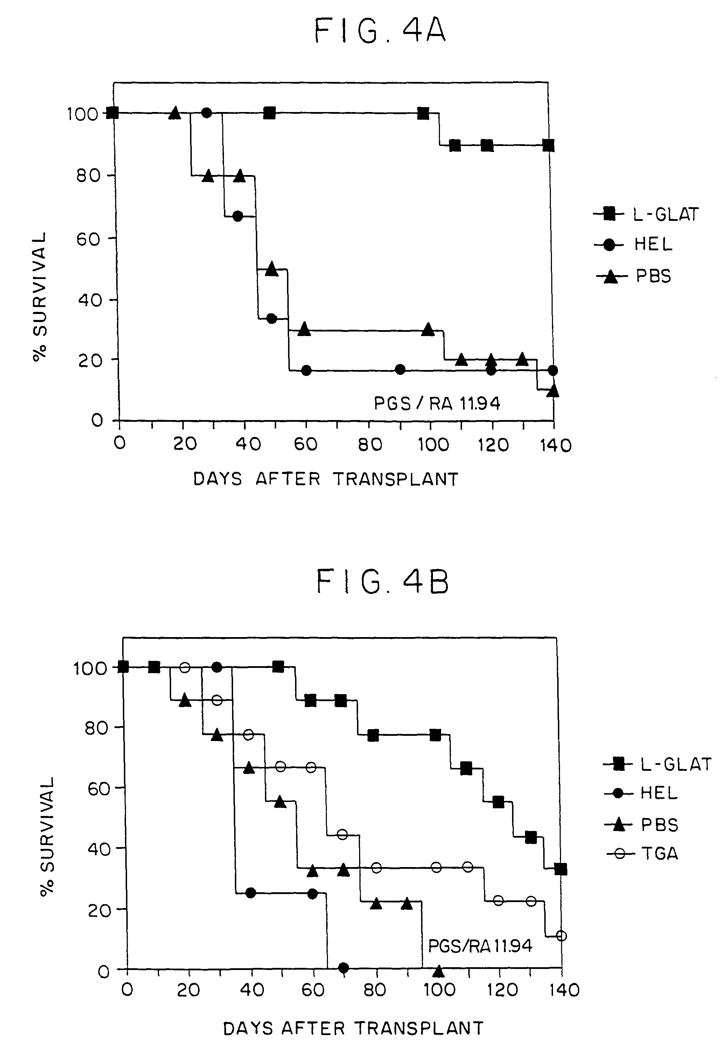

Figs. 4A-B show the effect on survival of BALB/c mice transplanted with bone marrow and spleen cells from B10.D2 mice after treatment with L-GLAT (Batch I), chicken egg lysozyme (HEL) or phosphate-buffered saline (PBS) (Fig. 4A) or with L-GLAT (Batch II), HEL, PBS or TGA polymer (Fig. 4B).

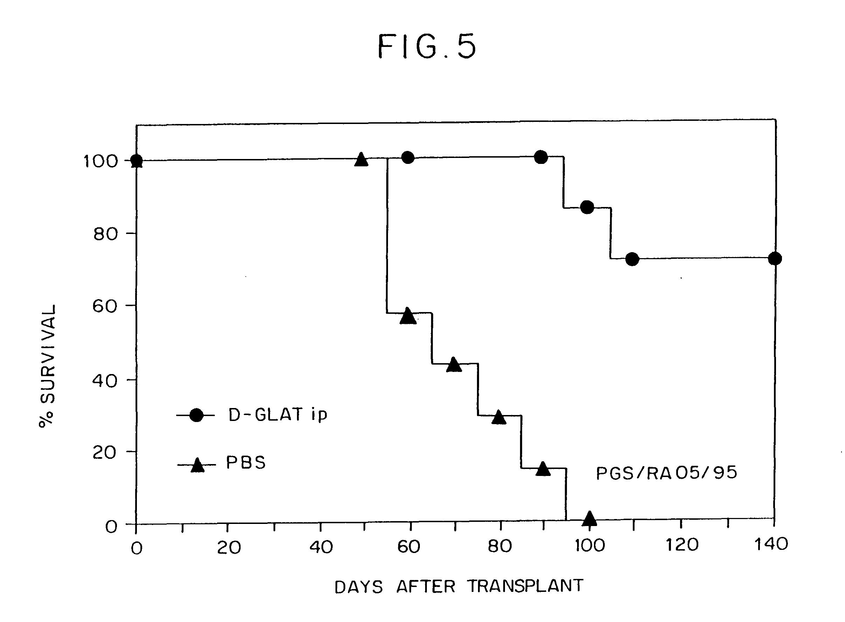

Fig. 5 shows the effect on survival of BALB/c mice transplanted with bone marrow and spleen cells from B10.D2 mice after treatment with D-GLAT or PBS.

### DETAILED DESCRIPTION OF THE INVENTION

The GLAT copolymers used in the present invention represent a novel therapeutic approach to treat human graft-versus-host disease for effective organ, particularly bone marrow, transplantation.

GLAT is a synthetic random basic copolymer analogous to Cop 1. Previous work has demonstrated two main and distinct mechanisms of action for Cop 1 which derive from its direct high-affinity binding to class II molecules of multiple murine and human alleles: a) cross-reactivity with myelin basic protein (MBP) at the humoral and cellular level leading to the induction of suppressive regulatory cells and b) competitive inhibition of the binding of myelin antigens to MHC, which may result in inhibition of specific effector T cell activation. The first mechanism has been shown to be most important for the prevention of EAE, a model in which a dominant antigen (MBP for H-2^{u} and PLP for H-2^{s} strains) leads to the induction phase of the autoimmune disease. On the contrary, GVHD is an alloimmune response induced by transplanted donor T cells in response to multiple minor antigens of the recipient. Some of the mHAg may occupy up to 10 % of binding sites whereas others may occupy as little as 0.001 % of MHC sites. Previous work has demonstrated that through the use of class-II binding allele-specific peptides of 11-14 amino acids, GVHD can be prevented. Prevention was found to be transient in some and permanent in others. This approach, however, has been limited by the need for allele-specificity and by the relatively short half-life of class-II peptides. GLAT, being of larger molecular weight, has been found to have an extended half-life in addition to its promiscuous but specific binding properties for multiple MHC class II alleles.

A series of *in vitro* experiments using T cell proliferative responses to major and minor histocompatibility antigens have shown that addition of GLAT inhibited the MLC in a dose-dependent inhibition in a variety of systems (H-2^{d} anti H-2^{u,s,k,b} or H-2^{b} anti H-2^{d}). Hen egg lysozyme (HEL) was used as specificity control. To exclude any toxic effect of GLAT on the responder cells, cells were successfully rechallenged with IL-2 in a subsequent assay. Furthermore, GLAT (1-100 µg/well) did not inhibit the IL-2 driven proliferation of the murine T cell clone G81. To investigate the role of T cell responses to minor antigens, a system was designed that allowed for antigen-presentation of mHAg on syngeneic antigen presenting cells (APC). GLAT reduced the proliferative responses to mHAg in a dose-dependent fashion. Maximum inhibition was found at a concentration of 80 µg/well. In an additional experiment, APC were harvested, preincubated for 24 hours with different concentrations of GLAT and washed before incubation with the responder cells. Prepulsing of the APC with GLAT reduced the subsequent proliferative response to mHAg, suggesting that GLAT inhibits antigen-presentation.

Ligation of MHC class II molecules has recently been shown to mediate apoptotic cell death in resting B lymphocytes under certain conditions. Incubation of spleen cells with GLAT for 48 or 72 hours did not result in apoptosis. These results were confirmed by FACS analysis using the Hoechst dye 33342.

Data from three consecutive experiments *in vivo* showed that administration of GLAT over a limited period after transplant prevented GVHD across minor histocompatibility barriers. Nine of 25 animals did not develop GVHD beyond the observation period of 140 days post transplant, whereas all control-treated mice (PBS or HEL) had developed GVHD by day 26. Furthermore, 12 of the GLAT treated mice showed a delayed onset of GVHD (range 32-112). GLAT improved disease severity as assessed by the severity score and by the mean body weight curves. GLAT resulted in a significantly improved survival from GVHD-related mortality (p<0.01) as compared to controls. The selected system of GVHD closely resembles the clinical setting of matched donor-recipient pairs. The murine system of B10.D2/nSnJ → BALB/c has been well elaborated. Both CD4⁺ and CD8⁺ T cell subsets are responsible for GVHD-induction in lethally irradiated BALB/c recipients, with CD4⁺ T cells being the predominant subset.

Data from the *in vitro* and *in vivo* studies are consistent with the concept that GLAT, by virtue of its ability to bind to multiple MHC class II molecules, inhibits antigen-presentation. Administration of GLAT over a limited time after transplant was successful in preventing the development of GVHD. Thus, GLAT represents a novel class of potent biological response modifiers for the prevention of GVHD across minor histocompatibility barriers.

The L-GLAT to be used according to the present invention may be prepared by methods known in the art, for example, the process disclosed in US Patent 3,849,550 and in Teitelbaum et al., 1971, wherein the N-carboxyanhydrides of tyrosine, alanine, γ-benzyl glutamate and ε-N-trifluoroacetyllysine are polymerized at ambient temperature in anhydrous dioxane with diethylamine as initiator, followed by deblocking of the γ-carboxyl group of the glutamic acid with hydrogen bromide in glacial acetic acid, and removal of the trifluoroacetyl groups from the lysine residues by 1M piperidine. L-GLAT with the required molecular weight profile of 4,000-12,000, preferably 6,000-8,000, can be obtained by methods known per se, for example, by chromatography of L-GLAT-containing high molecular weight species or by partial acid or enzymatic hydrolysis to remove the high molecular weight species with subsequent purification by dialysis or ultrafiltration.

D-Cop 1, an example of D-GLAT copolymer, has been found as non-cross-reactive with either the L-copolymer, Cop 1, or with the natural autoantigen myelin basic protein, and failed to exhibit any suppressive activity on EAE, indicating that it does not have a therapeutic value relevant to MS (Webb et al., 1976). According to the present invention, it was surprisingly found that D-GLAT binds as efficiently as L-GLAT to the MHC molecules, indicating its utility for prevention and treatment of GVHD.

D-GLAT and DL-GLAT copolymers may be prepared similarly to L-GLAT copolymers using D and D+L carboxyanhydrides, respectively, of the corresponding amino acids. An advantage in using a D-GLAT or DL-GLAT copolymer resides in the fact that such polymers metabolize much slower than the L-isomer, and thus a longer half-life and higher concentrations of the effective material can be achieved.

The pharmaceutical compositions of the present invention comprising a GLAT copolymer are prepared by conventional methods known in the art. Preferably, the composition is lyophilized and formed into an aqueous solution suitable for subcutaneous, intramuscular or intravenous injection. Typically, a pharmaceutical composition will comprise from 10 to 100 mg of L-GLAT in an unit dose. D-GLAT and DL-GLAT copolymers may be used in lower doses.

The GLAT copolymer may be used according to the invention for prevention and treatment of GVHD in all cases of organ transplantation that develop GVHD, but particularly in fetal thymus, and more particularly, in allogeneic bone marrow transplantation. To a patient under suitable conditioning regimen, the GLAT copolymer is administered from day -2 prior to the transplantation day, and then for another 60-100, at least 60, days, after the transplantation day. Other immunosuppressive drugs, such as cyclosporine, methotrexate and prednisone, may be administered with the GLAT copolymer.

The method of the invention is suitable for the prevention and treatment of GVHD in the course of bone marrow transplantation in patients suffering from diseases curable by bone marrow transplantation, including leukemias, such as acute lymphoblastic leukemia (ALL), acute nonlymphoblastic leukemia (ANLL), acute myelocytic leukemia (AML) and chronic myelocytic leukemia (CML), severe combined immunodeficiency syndromes (SCID), osteopetrosis, aplastic anemia, Gaucher's disease, thalassemia and other congenital or genetically-determined hematopoietic or metabolic abnormalities.

The invention will now be illustrated by the following non-limiting examples.

### EXAMPLES

### MATERIALS AND METHODS

### (a) Preparation of GLAT copolymers and controls

(i) L-GLAT was prepared by polymerization of the N-carboxyanhydrides of L-Ala, γ-benzyl-L-Glu, N,ε-trifluoroacetyl-L-Lys, and L-Tyr. The polymerization reaction was carried out at room temperature in anhydrous dioxane with diethylamine as initiator. Deblocking of the γ-carboxyl groups of the glutamic acid was carried out with hydrogen bromide in glacial acetic acid for 24 hours at room temperature, followed by removal of the trifluoroacetyl groups from the lysine residue by 1M piperidine. The end product is a mixture of acetate salts of random polypeptides with amino acid composition of Glu (1.4-2.1 residues), Lys (3.2-5.0 residues), Ala (4.0-6.6 residues), and Tyr (1 residue). Three L-GLAT batches were used in the experiments: Batch I consisting of a copolymer of molecular weight of about 6,000, with the amino acids in the molar ratio of about 1.7 Glu to 3.8 Lys to 4.9 Ala to 1.0 Tyr; Batch II, consisting of a copolymer of molecular weight of about 8,000, with the amino acids in the molar ratio of 1.8 Glu to 4.0 Lys to 6.0 Lys to 1.0 Tyr; and Batch III consisting of a copolymer of molecular weight of about 6,000 to 8,500 Da, with the amino acids in the molar ratio of 1.9 Glu to 4.7 Lys to 6.0 Ala to 1.0 Tyr.
(ii) D-GLAT was prepared by polymerization of the N-carboxyanhydrides of the D-amino acids D-Ala, γ-benzyl-D-Glu, N,ε-trifluoroacetyl-D-Lys, and D-Tyr in a residue molar ratio of 5.6 : 2.2 : 4.6 : 1.0 with an average molecular weight of approx. 29,000.
(iii) TGA is a random basic polymer of L-Tyr, γ-benzyl-L-Glu, and L-Ala in a residue molar ratio of 1.0: 1.2: 1.1. It was used as a negative control.
(iv) Hen egg-white lysozyme (HEL) was obtained from Sigma Chemical Company (St. Louis, MO).
(v) Peptides were synthesized by standard FMOC chemistry. All peptides were 95% to 99% pure, as determined by high-performance liquid chromatography, and were checked by amino acid analysis and mass spectroscopy. Sequences are given in three letter codes:
   MBP Ac1-11[4A], an acetylated N-terminal 1-11 peptide of myelin basic protein (MBP), with substitution of the original Lys residue at position 4 by Ala: Ac-Ala-Ser-Gln-Ala-Arg-Pro-Ser-Gln-Arg-His-Gly (SEQ ID NO:1);
   MBP 35-47, the epitope of MBP which is recognized in association with I-E^{u} : Thr-Gly-Ile-Leu-Asp-Ser-Ile-Gly-Arg-Phe-Phe-Ser-Gly (SEQ ID NO:2);
   KM-core extension peptide, based on the antigenic core sequence of ovalbumin 323-339: Lys-Met-Lys-Met-Val-His-Ala-Ala-His-Ala-Lys-Met-Lys-Met (SEQ ID NO:3);
   MBP 89-101: Val-His-Phe-Phe-Lys-Asn-Ile-Val-Thr-Pro-Arg-Thr-Pro (SEQ ID NO:4), was synthesized by t-butoxy-carbonyl chemistry.

### (b) Animals

B10.D2/nSnJ (H-2^{d}), CBA (H-2^{k}), C57BL/6 (H-2^{b}), B10.PL (H-2^{u}) and PL/J (H-2^{u}) mice were purchased from The Jackson Laboratories (Bar Harbor, ME); BALB/c (H-2^{d}) recipient mice were obtained from Simonsen Laboratories (Gilroy, CA). B10.S (H-2^{s}) were obtained from Dr. H.O. McDevitt's colony and bred in the animal facility of Stanford University.

### (c) Mixed lymphocyte cultures (MLC)

MLC were set up as previously described (Schlegel et al., 1995; Webb et al., 1990). Briefly, 2.5 × 10⁵ responder spleen cells were plated in flat-bottom 96 well microculture plates with 2.5, 5.0 or 10 × 10⁵ irradiated (30 Gy, Cs source) spleen stimulator cells in a final volume of 200 µl. GLAT or relevant controls were added at the indicated concentrations (10 - 100 µg/well). After 96 hours of incubation, cultures were pulsed with 1 µCi [³H]-thymidine for an additional 16 hours. Results are mean cpm from triplicate cultures.

### (d) Assessment of GVHD

Mice were followed up daily for 140 days after bone marrow transplantation for signs of GVHD. Disease severity was assessed by mortality, loss of body weight, and by the extent of macroscopic skin involvement scored on a cumulative severity scale from 0 (minimum) to 8 (maximum): head 1, neck 1, back (1/3, 2/3, 3/3) 1-3, front (1/3, 2/3, 3/3) 1-3 as previously described (Schlegel et al., 1994; Schlegel et al., 1995; Claman et al., 1985; Korngold et al., 1987; Hamilton 1987).

### (e) PCR analysis

Engraftment of donor bone marrow was documented by PCR amplification of a polymorphic microsatellite region within the murine IL-1β gene. Primer sequences are as follows: 5'-CCAAGCTTCCTTGTGCAAGTA-3' (SEQ ID NO:5) and 5'-AAGCCCAAAGTCCATCAGTGG-3' (SEQ ID NO:6) (Jacob et al., 1993). These sequences are available from EMBL/Gen-Bank/DDBJ database (Bethesda, MD) under the accession numbers: X78456 and X78457. Oligonucleotides were synthesized on a 391 DNA synthesizer (Applied Biosystems, Foster City, CA) and were purified. DNA was prepared from peripheral blood mononuclear cells 80-120 days after transplantation according to standard protocols (Schlegel et al., 1994). PCR conditions and amplification were as described previously (Schlegel et al., 1994).

### (f) Bone marrow transplant

For the induction of GVHD, 10 × 10⁶ bone marrow and 100 × 10⁶ spleen cells from B10.D2 mice were injected into lethally irradiated (8.0 Gy) BALB/c recipients. This regimen induces the most severe form of GVHD (Schlegel et al., 1995) and was selected for all experiments of this study. Recipient mice were 12 -13 weeks at the time of transplant. All experiments were performed according to U.S. federal and Stanford University guidelines.

### (g) Treatment

Recipient BALB/c mice were treated with the synthetic copolymer GLAT, phosphate-buffered saline (PBS), or with HEL. Based on previous studies with Cop 1 in EAE (Sela et al., 1990; Teitelbaum et al., 1971; Teitelbaum et al., 1973) and class II-binding competitor peptides in GVHD (Schlegel et al., 1994), the dosage of 600 µg per injection was selected, half of which was administered intraperitoneally (ip), whereas the other half was given subcutaneously (sc). Treatment of BALB/c recipient mice was initiated on day -1, followed by daily injections for the first five weeks starting on day 0 after transplant. The frequency of injections was tapered to three times per week for the subsequent two weeks and to two times per week for another two weeks and then discontinued. Throughout the experiment, copolymers and controls were administered once a week with incomplete Freund's adjuvant (IFA) ip as a depot dose (Schlegel et al., 1994). Treatment was discontinued 9 weeks after transplant.

### (h) Inhibition of minor histocompatibility (mH) antigen presentation

B10.D2 mice were injected i.p. with irradiated (20 Gy) BALB/c spleen cells 72 hours prior to the assay. B10.D2 spleen cells were harvested, depleted of RBC and used as irradiated (10 Gy) APC presenting BALB/c mH antigens. APC were plated at 2.5 × 10⁵ cells/well in 96 well round-bottom plates and incubated with 2.5 × 10⁵ nylon-wool-enriched responder T cells (> 88% CD3⁺ by FACS analysis) from naive B10.D2 mice in the presence or absence of increasing concentrations of GLAT (2.5 - 80 µg/well). After 96 hours of incubation, cells were pulsed with 1 µCi [³H]-thymidine for an additional 16 hours before harvesting. In a second experiment, irradiated APC were plated at 2.5 × 10⁵ cells/well in 96 well round-bottom plates, preincubated for 24 hours at 37°C with increasing concentrations of GLAT (2.5-80 µg/well) or with medium alone, and thereafter washed three times before the addition of 2.5 × 10⁵ nylon-wool-enriched responder T cells from naive B10.D2 mice. Culture medium was RPMI 1640, supplemented with 10% pre-screened fetal calf serum, 2mM glutamine, 50 µM 2-mercaptoethanol, 100 U/ml penicillin, and 100 µg/ml streptomycin.

### (i) Apoptosis

B10.D2 spleen cells (1 × 10⁶ per ml) were cultured in 24-well plates for 48 or 72 hours in the presence or absence of GLAT or HEL at the indicated concentrations. Irradiated (6.0Gy) B10.D2 thymocytes cultured for 24 hours served as the positive control (Sellins et al., 1987). Cells were collected, lysed (1mM EDTA, 50mM Tris (pH 7.5), 0.1 % NP-40) with 0.5 mg of proteinase K. DNA was extracted with phenol-chloroform two times and precipitated with ethanol (Schlegel et al., 1994). Aliquots of DNA were transferred to a 1.5 % agarose gel containing 0.05 µg/ml ethidium bromide. Presence or absence of DNA fragmentation was assessed as previously described (McConkey et al., 1988).

### EXAMPLE 1: Inhibition of mixed lymphocyte reaction by L-GLAT

Mixed lymphocyte reaction (MLR), the proliferative response of allogeneic lymphocytes when cultured together, is considered an *in vitro* model for the recognition phase of the GVHD reaction, and is part of the routine screening for bone marrow donors.

To investigate the feasibility of inhibition of MLR by L-GLAT, an MLR system was developed in which L-GLAT was tested for its ability to inhibit the proliferation of B10.PL (H-2^{u}) responder cells to stimulator cells of either the same (PL/J) or different (BALB/c) H-2 haplotype. For comparison, the inhibitory MBP Ac 1-11[4A], MBP 35-47, a combination of Ac 1-11[4A] + MBP 35-47, MBP 89-101 and KM-core peptides, described in Schlegel et al., 1994, were used in the MLR experiments.

As shown in Fig. 1A for stimulator cells of PL/J mice and in Fig. 1B for stimulator cells of BALB/c mice, L-GLAT (Batch I) significantly inhibited MLR across minor as well as major histocompatibility barriers. 63% and 77% inhibition could be obtained, respectively, when 1:1 ratio of responder to stimulator cells was used, while the MBP 89-101 and the KM-core peptides, which are specific to the H-2^{s} haplotype, did not induce any significant effect. The inhibition obtained by L-GLAT was similar (in response to minor histocompatibility antigen) or even higher (in response to major histocompatibility antigen) than the inhibition obtained with the combination of the two synthetic peptides Ac 1-11[4A] and MBP 35-47, which specifically bind to the class II molecules I-A^{u} and the I-E^{u}, respectively. The molar efficiency of L-GLAT of M.W. 6000 is even higher since the molecular weight of the synthetic peptides is 4-5 fold lower.

### EXAMPLE 2: Inhibition of MLC across major histocompatibility barriers

The effect of the synthetic copolymer L-GLAT (Batch III) on mixed lymphocyte cultures across major histocompatibility barriers was tested. T cell proliferation was assessed in six different MHC-disparate strain combinations. Data given in Figs. 2A-E are representative of five separate experiments with similar results. In all experiments, addition of L-GLAT (10-100 µg/well) resulted in a dose-dependent inhibition of the MLC. HEL showed no or only minimal inhibitory effect at all concentrations tested (Figs. 2A-E). 10-25 µg/well of L-GLAT was sufficient to achieve 50% inhibition of the proliferative responses. Maximum inhibition (100%) was obtained in all strain combinations tested. To exclude the possibility that higher concentrations of L-GLAT (50-100 µg/well) might be toxic to the responder cells, responder cells from background wells and from wells incubated with 25-100 µg/well of L-GLAT in the presence of stimulator cells for 72 hours were rechallenged in a subsequent assay with interleukin-2. As shown in Table 1, there was no difference between the secondary proliferative responses of the groups tested. Responder cells that had been incubated with stimulator cells in the presence of GLAT for 72 hours were equally responsive to IL-2 compared to non-treated cells.

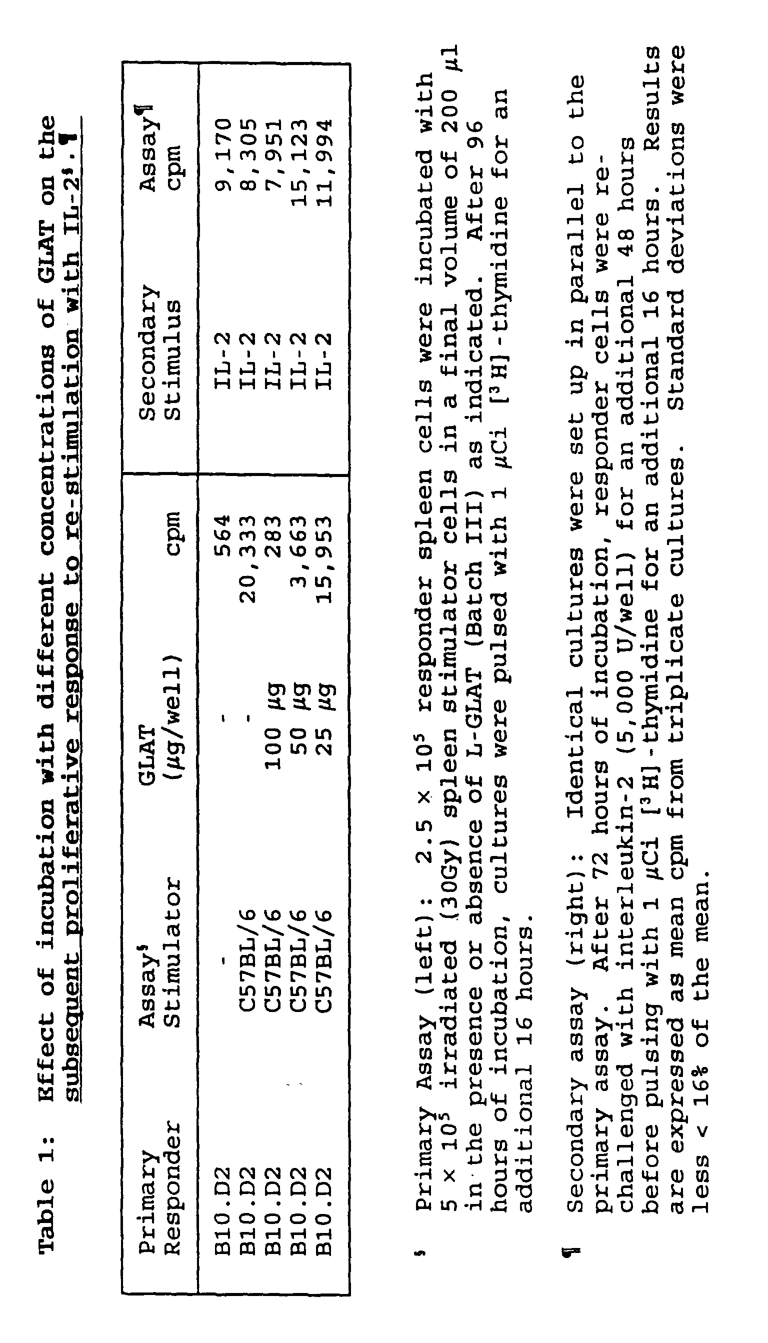

### EXAMPLE 3: Inhibition of proliferative responses to minor H antigens in vitro

Nylon wool-enriched T cells (>88% CD3⁺ cells by FACS analysis) from naive B10.D2 were incubated for 96 hours with syngeneic APC presenting minor BALB/c antigens as described in Materials and Methods. As shown in Table 2, L-GLAT (Batch III) inhibited the proliferative responses to mH antigens by in a dose-dependent fashion. Maximum inhibition was 100% at a concentration of 80 µg/well. To further elucidate the mechanism of inhibition, APC were preincubated for 24 hours with increasing concentrations of L-GLAT (2.5-80 µg/well), washed and plated thereafter with responder T cells. As shown in Table 2, preincubation with GLAT inhibited proliferative responses to minor H antigens, suggesting that GLAT inhibits presentation of minor antigens *in vitro.*

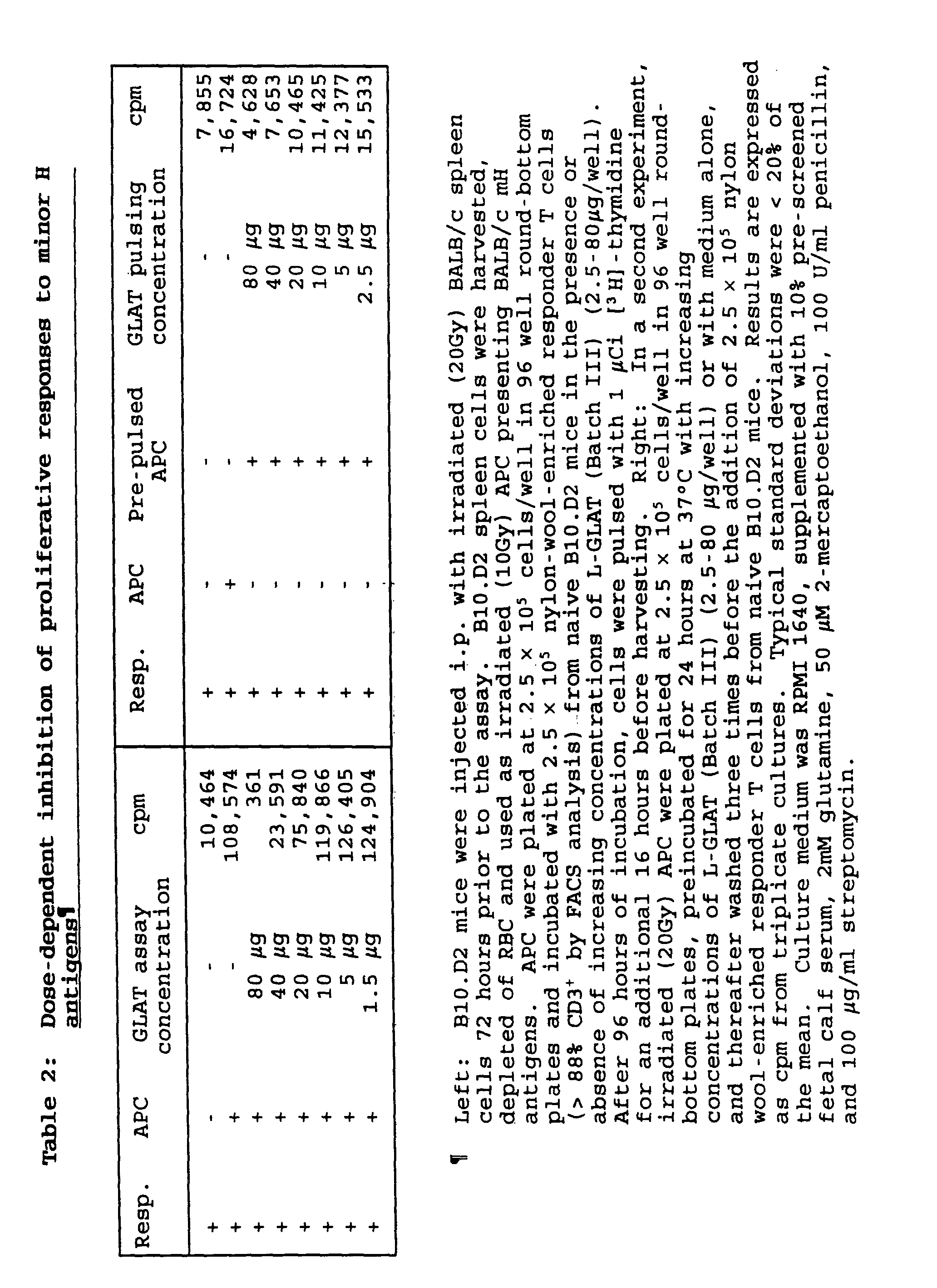

### EXAMPLE 4: Absence of apoptosis

Ligation of MHC class II molecules has recently been shown to mediate apoptotic cell death in resting B lymphocytes (Newell et al., 1993). To determine whether GLAT might induce apoptosis in class-II bearing cells (B cells and/or T cells) murine spleen cells were incubated with increasing concentrations of L-GLAT (Batch III) or HEL-control for 48 or 72 hours. Aliquots of cells were processed for DNA extraction and DNA was visualized by gel electrophoresis. L-GLAT did not induce apoptosis at any of the concentrations tested.

### EXAMPLE 5: Bone marrow transplantation in mice, induction of GVHD and treatment with L-GLAT

(i) Initial titration study. For the induction of GVHD across minor histocompatibility barriers, an initial titration study was performed by transplanting 10 × 10⁶ B10.D2 bone marrow cells and increasing amounts (10-100 × 10⁶) of B10.D2 spleen cells into lethally irradiated (8.0 Gy) 12-13 week old BALB/c recipients. The regimen of infusing 10 × 10⁶ bone marrow cells and 100 × 10⁶ spleen cells resulted in the most severe form of GVHD, and was selected for all subsequent experiments.
(ii) Effect of L-GLAT treatment on the incidence. onset and severity of GVHD. Recipient mice were pretreated with 600 µg of L-GLAT (Batch III) or with the respective controls (PBS, HEL) on day -1. For the first five weeks after transplant, mice were injected daily as outlined in Materials and Methods, followed by a tapering schedule over an additional four weeks. Data from three consecutive experiments are summarized in Figures 3A-D. Administration of L-GLAT significantly reduced the overall incidence of GVHD (as determined by typical skin changes and weight loss) from 100% (26/26, 10/10) in control mice to 12% (3/25) (p<0.001) in L-GLAT-treated animals on day 30 after transplant, and from 100% in controls to 12/25 (48%) on day 70 after transplant (p<0.02). Figure 3A depicts the onset of GVHD in individual mice of the different experimental groups. In 12/25 animals treated with L-GLAT the onset of GVHD was delayed with a range of 32-112 days after transplant (median of 73 days) as compared to control mice treated with either PBS (median onset of 21 days) or HEL (median onset 22 days). Nine of 25 animals treated with the copolymer L-GLAT did not develop any signs of GVHD beyond the observation period of 140 days after transplant (Fig. 3A). Furthermore, treatment with GLAT improved overall disease severity as gauged by the disease severity score (Fig. 3B) and by mean body weight curves of transplanted animals (Fig. 3D).
   Similar results were obtained with Batches I and II of L-GLAT.
(iii) Effect of L-GLAT treatment on survival. Treatment with L-GLAT (Batch III) improved long-term survival from lethal graft-versus-host disease. As shown in Fig. 3C, 14/25 (56%) of experimental mice survived more than 140 days after transplant as compared to 2/26 of PBS treated or to 1/10 of HEL treated control mice (p<0.01). Treatment with HEL did not improve long-term survival.
   Similar experiments were performed substituting L-GLAT Batch I (Fig. 4A) or Batch II (Fig. 4B). As shown in Figs. 4A-B, treatment with L-GLAT for the first nine weeks after bone marrow transplantation improved long-term survival from lethal GVHD. Fig. 4A shows that 9 out of 10 (90%) experimental mice survived more than 140 days after transplant as compared to only 1 out of 10 for the PBS control or 1 out of 6 for the HEL-treated control mice. In these figures, means are stated as overall means of the respective groups. GVHD-related mortality. MST: median survival time (in days). Statistical analysis: Incidence of GVHD by χ² distribution: Experiment 1 (Fig. 4A): * p < 0.01, ** p < 0.001, L-GLAT compared to PBS or HEL. Experiment 2 (Fig. 4B): π p < 0.02, L-GLAT compared to PBS, TGA or HEL.
   Similar results were obtained with L-GLAT Batch II, as shown in Fig. 4B.
(iv) Documentation of engraftment. PCR analysis was performed 100 days post-transplant as described in Materials and Methods, section (e), to document long-term engraftment of allogeneic bone marrow cells. DNA polymorphism based on length variation in tandem repeat sequences of a microsatellite in the murine IL-1 gene was used as marker to differentiate between donor-derived (B10.D2/nSnJ) and recipient (BALB/c) peripheral blood mononuclear cells. Long-term engraftment of donor-derived cells, i.e., complete chimerism, was demonstrated in allogeneic mice by PCR analysis irrespective of the treatment received.

### EXAMPLE 6: Bone marrow transplantation in mice, induction of GVHD and treatment with D-GLAT

(i) Initial titration study was carried out as in Example 5(i).
(ii) Effect of D-GLAT treatment on the incidence, onset and severity of GVHD. Recipient mice were treated with 60 µg of D-GLAT or PBS daily for the first five weeks after transplant followed by a tapering schedule over an additional four weeks. The dosage of 60 µg/injection was selected based on the prolonged half-life of D-GLAT and was administered intraperitoneally (ip). Treatment was initiated on day -1 and after five weeks the frequency of injections was tapered to three times per week for the following two weeks and to two times per week for another two weeks. Once a week the copolymer D-GLAT was administered with incomplete Freund's adjuvant (IFA) ip as a depot dose. Treatment was discontinued after 9 weeks.
   The results are shown in Table 3. D-GLAT treatment reduced the overall incidence of GVHD after allogeneic bone marrow transplantation from 100% (7/7) in control mice to 43% (3/7) in D-GLAT-treated animals on day 30. Two of seven animals treated with D-GLAT did not develop any signs of GVHD beyond the observation period of 140 days after transplant. Furthermore, treatment with D-GLAT improved overall disease severity as gauged by the mean disease severity score (Table 3).
(iii) Effect of D-GLAT treatment on survival. Treatment with D-GLAT improved long-term survival from lethal graft-versus-host disease. As shown in Fig. 3, 5/7 (71.4%) of experimental mice survived more than 140 days after transplant as compared to 0/7 of PBS-treated control mice.

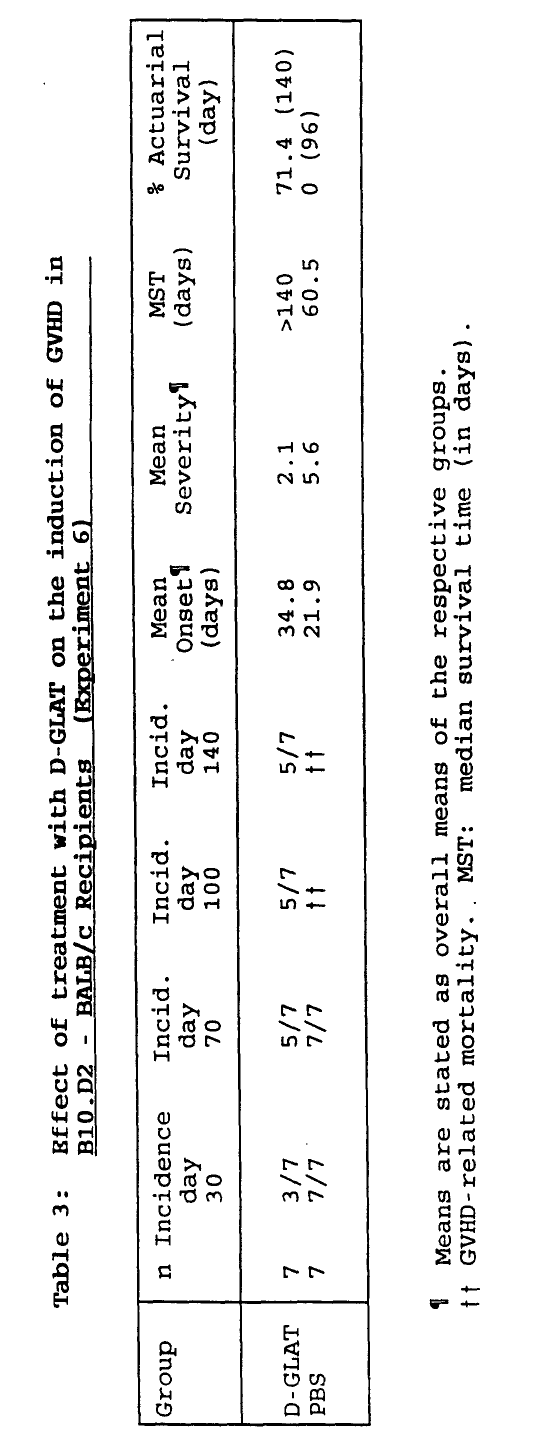

### EXAMPLE 7: Prevention of GVHD in humans

(i) Patients. A protocol is established for patients aged 60 years or less and eligible for allogeneic bone marrow transplantation from histocompatible sibling donors for acute non-lymphoblastic leukemia or acute lymphoblastic leukemia not in first remission, chronic myelogenous leukemia not in chronic phase or relapsed patients with non-Hodgkin's lymphoma. Without bone marrow transplantation, these patients have expected survival measured in months. The benefits for these subjects is the use of high dose therapy in curing their disease and the potential prophylaxis against morbidity associated with allogeneic bone marrow transplantation, such as GVHD.
(ii) Conditioning Regimen. Patients are submitted to a conditioning regimen comprising fractionated total body irradiation by administering 120 cGy per fraction 2 to 3 times daily over 4 days (days -8 to -5) to a total dose of 1200 cGy, a multiple drug treatment including, e.g., etoposide (60 mg/kg) over 4 hours at day -4, cyclophosphamide (60 mg/kg) over 1 hour at day -2 , antibacterial agents, and standard prophylaxis agents against GVHD, e.g., cyclosporine, methotrexate and prednisone.
(iii) GVHD prophylaxis. GLAT copolymer is administered to the patient subcutaneously (sc), intramuscularly (im) or intravenously (iv) as a twice daily injection at a dosage of 1-500 mg twice daily. The treatment starts at day -2 before and is continued until day +60 after the allogeneic bone marrow transplantation (BMT). This day is chosen since nearly all of the acute GVHD that may occur, will do so by day +40.
   Standard prophylaxis against GVHD with cyclosporine and prednisone is also continued. Cyclosporine is administered until the patient is able to sustain oral caloric intake and has no evidence of gastrointestinal toxicity (usually around the end of the first month, 4th week following BMT), at a dose from 1.5 to 5 mg/kg iv twice daily, by infusion in the first 35 days and then orally (per os) until the end of the treatment (day +180). Serum samples are obtained and, if necessary, the drug concentration is adjusted to prevent drug-related toxicities. The aim is for a level of cyclosporine between 200-500 ng/ml. Methylprednisolone is administered iv until patients can be switched to oral (p.o.) prednisone. For example, it is administered iv at a dose of 0.25 mg/kg - 0.5 mg/kg from day +7 to +28 and p.o. at a dose of 0.4 mg/kg - 0.1 mg/kg until the end of the treatment (day +180).
   The first phase of the treatment is to establish engraftment. If engraftment is established, the post-transplantation immunosuppression is then stepwise decreased. The first step is to stop the use of prednisone. If no GVHD occurs, then the use of cyclosporine is stopped (see statistical analysis). At that time only the GLAT copolymer is used as the immunosuppressive regimen.
(iv) Statistical analysis. Current engraftment success rate of bone marrow is close to 100% with a rate of about 50% at 20 days for patients satisfying the eligibility criteria of this protocol. Among these patients, the rate of relapse depends on the disease and remission status. The time to relapse curve is well approximated by an exponential curve over this interval. Both the engraftment rate at 20 days and the relapse rate are monitored statistically as the data become available on other patients treated by the same protocol.
   The engraftment rate at 20 days provides the basis for stopping early to avoid putting more patients at risk than necessary if the stem cells take longer to engraft than anticipated. The binary endpoint of success by the 20th day is monitored by sequential test, using 20 as the hypothesized median based on past experience, and using a boundary for inferiority fixed to provide a Type I risk of 5% (one sided) and a degree of conservatism midway between constant p level and the O'Brien-Fleming approach.
   As the treatment proceeds, the data on relapse is also monitored, using the time to relapse as the endpoint and including all patients under treatment, whether engraftment is successful at the 20th day or not.

### REFERENCES

Aharoni, R., et al., (1993) Eur. J. Immunol., 23:17-25.
Blazar, B.R., et al., (1994) J. Immunol., 152:3665-74.
Bornstein, M.B., et al., (1990) "Clinical trials of Cop 1 in multiple sclerosis", in Handbook of Multiple Sclerosis, ed. Cook S.D. Marcel Dekker, Inc., p.469.
Claman, H.N., et al., (1985) Cell. Immunol., 94:73-84.
den Haan, J.M., et al., (1995) Science, 268:1476-1480.
Fridkis-Hareli, M., et al., (1994) "Direct binding of myelin basic protein and synthetic copolymer 1 to class II major histocompatibility complex molecules on living antigen-presenting cells - specificity and promiscuity", PNAS, 91:4872-76.
Fridkis-Hareli, M., et al., (1995) Cell Immunol., 168:229-236.
Griem, P., et al., (1991) Cell, 65:633-640.
Hamilton, B.L., (1987) J. Immunol., 139:2511-2555.
Ishioka, G.Y., et al., (1994) J. Immunol., 152:4310-4319.
Jacob, C.O., et al., (1993) "DNA polymorphism in cytokine genes based on length variations in simple-sequence tandem repeats", Immunogenetics, 38:251.
Johnson, P.K., et al., (1994) "Cop 1 positive results - a phase III trial in relapsing remitting MS", 11 Annual Meeting A.N.A. 1994.
Korngold, R., et al., (1987) J. Exp. Med., 165:1552-1564.
McConkey, D.J., et al., (1988) Science, 242:256-259.
Nestel, F.P., et al., (1992), J. Exp. Med., 175:405-13.
Newell, M.K., et al., (1993) PNAS, 90:10459-63.
Roopenian, D.C., et al., (1993), Immunogenetics, 38:131-140.
Rus, et al., (1995), J. Immunol., 155:2396-2406.
Samson, M.F., et al., (1995) J. Immunol., 155:2737-2746.
Schlegel, P.G., et al., (1994) "Prevention of Graft-Versus-Host Disease by Peptides Binding to Class II Major Histocompatibility Complex Molecules", Blood, 84:2802-10.
Schlegel, P.G., et al., (1995) J. Immunol., 155:3856-3865.
Sela, M., et al., (1990) Bull. Inst. Pasteur (Paris), 88:303-314.
Sellins, K.S., et al., (1987) J. Immunol., 139:3199-3206.
Speiser, D.E., et al., (1990), PNAS, 87:2021-2025.
Teitelbaum, D., et al., (1971) "Suppression of experimental allergic encephalomyelitis by a synthetic polypeptide". Eur. J. Immunol., 1:242-48.
Teitelbaum, D., et al., (1973) "Suppression by several synthetic polypeptides of experimental allergic encephalomyelitis induced in guinea pigs and rabbits with bovine and human basic encephalitogen", Eur. J. Immunol., 3:273-279.
Teitelbaum, D., et al., (1974a) "Suppression of experimental allergic encephalomyelitis in rhesus monkeys by a synthetic basic copolymer", Clin. Immunol. Immunopathol., 3:256.
Teitelbaum, D., et al., (1974b) "Suppression of experimental allergic encephalomyelitis in baboons by Cop 1", Israel J. Med. Sci., 13:1038.
Teitelbaum, D., et al., (1988) PNAS, 85:9724-28.
Wallny, P.J., et al., (1990), Nature (London), 343:275-278.
Wang, W., et al., (1995) Science, 269:1588-1590.
Webb C., et al., (1976) Immunochemistry 13:333.
Webb, S., et al., (1990) Cell, 63:1249-1256.

## Claims

1. Use of a synthetic random copolymer of average molecular weight 4,000-12,000, herein referred to as GLAT copolymer, said GLAT copolymer consisting of glutamic acid (Glu), lysine (Lys), alanine (Ala) and tyrosine (Tyr) residues in a relative molar ratio of 1.4-2.1 parts of Glu to 3.2-5.0 parts of Lys to 4.0-6.6 parts of Ala to 1.0 part of Tyr for the preparation of a pharmaceutical composition for preventing and treating graft-versus-host disease in a patient in the course of bone marrow or organ transplantation.

2. Use according to claim 1, wherein the GLAT copolymer has an average molecular weight of 5,500-10,000.

3. Use according to claim 1, wherein the GLAT copolymer has an average molecular weight of 6,000-8,000.

4. Use according to any one of claims 1 to 3, wherein all amino acid residues in the copolymer have the L configuration, herein referred to as L-GLAT copolymer.

5. Use according to any one of claims 1 to 3, wherein all amino acid residues in the copolymer have the D configuration, herein referred to as D-GLAT copolymer.

6. Use according to any one of claims 1 to 3, wherein some of the amino acid residues of the copolymer have the L configuration and the other amino acid residues have the D configuration, herein referred to as DL-GLAT copolymer.

7. Use according to claim 4, wherein said L-GLAT copolymer has an average molecular weight of 6,000 and a relative molar ratio of 1.7 parts Glu to 3.8 parts Lys to 4.9 parts Ala to 1.0 parts Tyr.

8. Use according to claim 4, wherein said L-GLAT copolymer has an average molecular weight of 8,000 and a relative molar ratio of 1.8 parts Glu to 4.0 parts Lys to 6.0 parts Ala to 1.0 parts Tyr.

9. Use according to claim 4, wherein said L-GLAT copolymer has a relative molar ratio of 1.9 Glu to 4.7 Lys to 6.0 Ala to 1.0 Tyr.

10. Use according to claim 1 wherein the pharmaceutical composition is in a form suitable for intravenous, intramuscular or subcutaneous administration.

## Patentansprüche

1. Verwendung eines synthetischen Zufalls-Copolymers mit einem durchschnittlichen Molekulargewicht von 4.000 - 12.000, im Folgenden als GLAT-Copolymer bezeichnet, wobei das GLAT-Copolymer aus Glutaminsäure(Glu)-, Lysin(Lys)-, Alanin(Ala)- und Tyrosin(Tyr)-Resten, in einem relativen molaren Verhältnis von 1,4 - 2,1 Teilen Glu zu 3,2 - 5,0 Teilen Lys zu 4,0 - 6,6 Teilen Ala zu 1,0 Teil Tyr besteht, für die Herstellung eines Arzneimittels zur Prävention und Behandlung von Graft-versus-Host-Erkrankungen in einem Patienten in der Folge von Knochenmarks- oder Organtransplantationen.

2. Verwendung nach Anspruch 1, wobei das GLAT-Copolymer ein durchschnittliches Molekulargewicht von 5.500 - 10.000 hat.

3. Verwendung nach Anspruch 1, wobei das GLAT-Copolymer ein durchschnittliches Molekulargewicht von 6.000 - 8.000 hat.

4. Verwendung nach einem der Ansprüche 1 bis 3, wobei alle Aminosäurereste in dem Copolymer die L-Konfiguration besitzen, im Folgenden als L-GLAT-Copolymer bezeichnet.

5. Verwendung nach einem der Ansprüche 1 bis 3, wobei alle Aminosäurereste in dem Copolymer die D-Konfiguration haben, im Folgenden als D-GLAT-Copolymer bezeichnet.

6. Verwendung nach einem der Ansprüche 1 bis 3, wobei einige der Aminosäurereste des Copolymers die L-Konfiguration und andere Aminosäurereste die D-Konfiguration haben, im Folgenden als DL-GLAT-Copolymer bezeichnet.

7. Verwendung nach Anspruch 4, wobei das L-GLAT-Copolymer ein durchschnittliches Molekulargewicht von 6.000 und ein relatives molares Verhältnis von 1,7 Teilen Glu zu 3,8 Teilen Lys zu 4,9 Teilen Ala zu 1,0 Teil Tyr hat.

8. Verwendung nach Anspruch 4, wobei das L-GLAT-Copolymer ein durchschnittliches Molekulargewicht von 8.000 hat und ein relatives molares Verhältnis von 1,8 Teilen Glu zu 4,0 Teilen Lys zu 6,0 Teilen Ala zu 1,0 Teil Tyr hat.

9. Verwendung nach Anspruch 4, wobei das L-GLAT-Copolymer ein relatives molares Verhältnis von 1,9 Glu zu 4,7 Lys zu 6,0 Ala zu 1,0 Tyr hat.

10. Verwendung nach Anspruch 1, wobei das Arzneimittel in einer Form vorliegt, die geeignet ist für die intravenöse, intramuskuläre oder subkutane Verabreichung.

## Revendications

1. Utilisation d'un copolymère statistique de synthèse ayant un poids moléculaire moyen compris entre 4000 et 12000, appelé ici copolymère GLAT, ledit copolymère GLAT étant constitué de résidus d'acide glutamique (Glu), de lysine (Lys), d'alanine (Ala) et de tyrosine (Tyr) avec un rapport molaire relatif de 1,4-2,1 parties de Glu pour 3,2-5,0 parties de Lys pour 4,0-6,6 parties d'Ala pour 1,0 partie de Tyr, pour la préparation d'une composition pharmaceutique destiner à prévenir et traiter une affection due à une réaction de greffe chez un patient , dans le cadre d'une greffe de moelle osseuse ou d'une transplantation d'organe.

2. Utilisation selon la revendication 1, dans laquelle le copolymère GLAT a un poids moléculaire moyen compris entre 5500 - 10000.

3. Utilisation selon la revendication 1, dans laquelle le copolymère GLAT a un poids moléculaire moyen compris entre 6000 - 8000.

4. Utilisation selon une quelconque des revendications 1 à 3, dans laquelle tous les résidus d'acides aminés dans le copolymère ont la configuration L, ce qui sera désignée ici par copolymère L-GLAT.

5. Utilisation selon une quelconque des revendications 1 à 3, dans laquelle tous les résidus d'acides aminés dans le copolymère ont la configuration D, désignée ici par copolymère D-GLAT.

6. Utilisation selon l'une quelconque des revendications 1 à 3, dans laquelle certains des résidus d'acides aminés du copolymère ont la configuration L et les autres résidus d'acides aminés ont la configuration D, ce qui sera désigné ici par copolymère DL-GLAT.

7. Utilisation selon la revendication 4, dans laquelle ledit copolymère L-GLAT a un poids moléculaire moyen de 6000 et un rapport molaire relatif de 1,7 parties de Glu pour 3,8 parties de Lys pour 4,9 parties d'Ala pour 1,0 partie de Tyr.

8. Utilisation selon la revendication 4, dans laquelle ledit copolymère L-GLAT a un poids moléculaire moyen de 8000 et un rapport molaire relatif de 1,8 parties de Glu pour 4,0 parties de Lys pour 6,0 parties d'Ala pour 1,0 partie de Tyr.

9. Utilisation selon la revendication 4, dans laquelle ledit copolymère L-GLAT a un rapport molaire relatif de 1,9 parties de Glu pour 4,7 parties de Lys pour 6,0 parties d'Ala pour 1,0 partie de Tyr.

10. Utilisation selon la revendication 1, dans laquelle la composition pharmaceutique est sous une forme appropriée pour une administration intraveineuse, intramusculaire ou sous-cutanée.
